# EUROPEAN PATENT APPLICATION

(11) **EP 0 610 110 A1**
(43) Date of publication of application: **10.08.1994**
(21) Application number: 94400116.3
(22) Date of filing: 19.01.1994
(51) Int. Cl.: A61M 16/00

(54) **Mouth-to-mouth resuscitator**

(30) Priority: 01.02.1993 IL 10457193
(71) Applicant: RABINTEX INDUSTRIES LTD., Bnei-Brak 51201 (IL)
(72) Inventor: Alon, Uri, Hadar-Am 42935 (IL); Reshef, Yaron, Pardesia 42815 (IL)
(74) Representative: Dubois-Chabert, Guy

(57) **Abstract**

A mouth-to-mouth resuscitator for use by a rescuer in order to force air into a victim while avoiding direct mouth-to-mouth contact between the rescuer and the victim is provided. The device comprises a vertically extending hollow body (10) having a first, top (12) and a second, bottom (14) portions, a first portion (12) has a mouth piece at its upper end and has a substantially cross-sectional shape, and the second portion (14) has an air outlet at its lower end and is adapted for insertion into the victim's mouth. The hollow body (10) has a one-way valve assembly (16) between the first (12) and the second (14) portions which permits air passage only from the direction of the first portion (12) into the second portion (14). The above assembly (16) has a rigid valve seat (24) which has a central portion which allows air passage and a flexible valve member (26) below the valve seat (24) which is upwardly biased against the valve seat (24). The device further comprises a pliable sheet (18) which extends laterally from around the hollow body (10), at about the zone thereof which is between the first (12) and second (14) portions of the hollow body (10) of the sheet (18), separated between the mouth area of the victim and the mouth of the rescuer.

## Description

### FIELD OF THE INVENTION

The invention concerns a mouth to mouth resuscitator for use by a rescuer in order to force air into a victim while avoiding direct mouth-to-mouth contact between the rescuer and the victim.

### BACKGROUND OF THE PRESENT INVENTION AND PRIOR ART

Mouth-to-mouth resuscitation is one of the most important methods in reviving non-breathing victims, particularly by untrained people outside the hospital. It is thus, common practice for first aid personnel, policemen, firemen and the like, to carry with them a mouth-to-mouth resuscitator.

In recent years, with the spread of some very severe infectious diseases, such as AIDS (acquired immuno deficiency syndrome) or Hepatitis B, performance of mouth-to-mouth resuscitation in general even by the use of mouth-to-mouth resuscitators became problematic in view of the possibility that the victim may be a carrier of such an infectious disease and thus the rescuer may face the risk of becoming infected. The presence of infectious particles in exhaled air or in orally emitted body fluids such as in exhaled saliva or vomit, is known for some infectious diseases and is believed to be present in others. As a result people are now very reluctant to even come into contact with exhaled air or body fluids of unfamiliar individuals, *a fortiori* to inhale such exhaled air or orally emitted fluids. Consequently, there have been numerous reports of cases in which non-breathing victims were left untreated until the arrival of medical personnel equipped with resuscitation instruments, this critical delay in resuscitation resulting in the victim's death.

U.S. Patent 4,819,628, discloses a resuscitator intended to solve the above-noted problems associated with mouth-to-mouth resuscitation. The resuscitator in accordance with this patent comprises a rigid tube fixed with a one-way valve and extending from the tube is a flexible sheet intended to physically shield the rescuer from the victim. The one-way valve consists of a flexible sleeve opened at the upper end and having a bottom end which is normally closed by the biasing force of a strip spring made of rigid and flexible material secured to the bottom end of the sleeve.

Another mouth-to-mouth resuscitator meant to solve some of the above-noted problems has been disclosed in U.S. Patent 4,697,587. The resuscitator of this patent includes a hollow body fitted at its upper end with a mask intended to cover the victim's mouth during resuscitation and has a flapper type valve inside the hollow body. This flapper type valve closes when the victim exhales, thus protecting the rescuer from inhaling the bulk of the victim's exhaled air or emitted fluids. This resuscitator further has an escape passage that by-passes the flapper valve and allows exhalation of breath as well as other body fluids emitted from the victim's mouth.

From analysis performed during development of the present invention, it has been realized that none of the above prior art resuscitators provide a sufficiently satisfactory protection for the rescuer against contraction of infection from the victim. In the case of the resuscitator of U.S. Patent 4,819,628, very small quantities of air carrying with them solid microparticles or tiny aerosol particles may escape through the strip spring into the space enclosed by the flexible sleeve and consequently be inhaled by the rescuer. Additionally, in the case of the resuscitator of U.S. Patent 4,697,587, the flapper valve also allows escape of some of the victim's air, e.g. in the case of back pressure during coughing of the victim and furthermore, the air or fluid escaping through the escape passage may also be inhaled by the rescuer or otherwise come into contact with him.

It should be noted in this connection that in various infectious diseases, contact with even very few infectious particles may give rise at times to the development of infectious diseases, and accordingly in order to provide adequate protection, a resuscitator should be designed for protection against inhalation of even minute particles, solid microparticles or tiny aerosol particles, suspended in the exhaled air.

### SUMMARY OF THE INVENTION

It is the object of the present invention to provide a mouth-to-mouth resuscitator which provides a very high degree of protection of the rescuer from inhaling the victim's exhaled air and various tiny solid or aerosol particles originating in the victim, or otherwise contacting body fluids emitted through the victim's mouth.

It is another object of the invention to provide a mouth-to-mouth resuscitator which allows the rescuer to approach the victim for the performance of the resuscitation, from various directions, e.g. from the direction of the victim's head or from the victim's side.

In accordance with the present invention a novel mouth-to-mouth resuscitator is provided which has the following very important features:
(i) it has a valve assembly of a kind which practically avoids any passage of fluids or air from the victim to the rescuer and allowing only a one-way passage in the other direction;
(ii) the hollow body or duct which directs the air exhaled by the rescuer into the victim's mouth is divided by a valve assembly into two portions, a bottom portion facing the victim and a top portion facing the rescuer and fitted with a mouth piece at its upper end; the bottom portion has a larger volume and seeing that during resuscitation it is filled by the rescuer's exhaled air, it serves as a buffer zone which almost totally prevents any of the victim's exhaled air or microparticles originating from the victim from reaching the valve assembly; and
(iii) the mouth piece is of a kind allowing a firm grip by the mouth of the rescuer virtually independent of the direction from which the rescuer approaches the victim, i.e. whether from the side or from the direction of the victim's head.

In accordance with the present invention there is thus provided a mouth-to-mouth resuscitator device for use by a rescuer in order to force air into a victim while avoiding direct mouth-to-mouth contact between the rescuer and the victim, said device comprising:
a vertically extending hollow, open ended body adapted to transmit air exhaled from the rescuer to the victim and having a first, top and a second, bottom portions, the first portion has an essentially circular mouth piece, and the second portion is adapted to be inserted into the victims mouth;
a one-way valve assembly contained in said hollow body and dividing it into said first and second portions and permitting air passage only from the first portion into the second portion, the valve assembly comprising a rigid valve seat and a flexible valve member below the valve seat, the valve seat having a central portion allowing free air passage, and the valve member being essentially disk-shaped and being upwardly biased against the valve seat so that peripheral portions of the valve member presses against peripheral portions of the valve seat to form an air-tight contact; and
a pliable sheet extending laterally from around said hollow body from about the zone thereof being between said first and said second portions, said sheet separating between the mouth area of the victim and the mouth of the rescuer.

In accordance with a preferred embodiment of the present invention the second member is sufficiently long to serve as a buffer zone preventing solid microparticles or aerosol particles coming from the victim to reach the valve assembly.

In accordance with another preferred embodiment of the present invention, the central portion of the valve seat comprises spokes radially extending from a centrally bored hub-like member in the middle of said central portion, the valve seat comprising also an annular ridge protruding downward from a bottom face at the periphery thereof. The valve member in accordance with this preferred embodiment is made of an elastic material such as silicone rubber and has a diameter somewhat larger than that of the annular ridge. The valve member has a central anchoring leg adapted to be inserted in the bore in said hub-like member, and brought into a locking position in which the valve member is anchored in said valve assembly by said anchoring leg and a peripheral portion of the valve member is biased against the peripheral annular ridge of the valve seat.

### BRIEF DESCRIPTION OF THE DRAWINGS

**Fig. 1** shows a perspective view of a mouth-to-mouth resuscitator in accordance with one embodiment of the invention with a portion cut away to show internal structure;
**Fig. 2** is a side elevation of the resuscitator from the direction of arrow 2 in Fig. 1;
**Fig. 3** is a side elevation of the resuscitator from the direction of arrow 3 in Fig. 1;
**Fig. 4** is a cross-section through lines 4-4 in Fig. 3;
**Fig. 5** shows a longitudinal cross-sectional view of a resuscitator in accordance with another embodiment of the invention;
**Fig. 6** shows a cross-section through a valve member;
**Fig. 7** is a view from the direction of arrow 7 in Fig. 6;
**Fig. 8** is a cross-sectional view through the first portion of the resuscitator of Fig. 6; and
**Fig. 9** is a view from the direction of arrow 9 in Fig. 8, with the valve member removed.

### DESCRIPTION OF SPECIFIC EMBODIMENTS

The invention will now be illustrated by reference to specific embodiments which will be described in the following with occasional reference to the annexed drawings. The specific embodiments described herein should by no means be construed as limiting, and it will no doubt be appreciated that these specific embodiments are an example only of a much wider scope of the invention, as defined herein.

The terms *"upper"* and *"lower"* will be used to refer to portions of the resuscitator in relation to the manner of its use in the resuscitation of a non-breathing victim. Such a victim is, as a rule, placed on its back and a portion of the resuscitator, to be referred to herein as the *"lower portion"* is inserted into its mouth, and then air is forced into the victim through the resuscitator's mouth piece which is in its *"upper portion".*

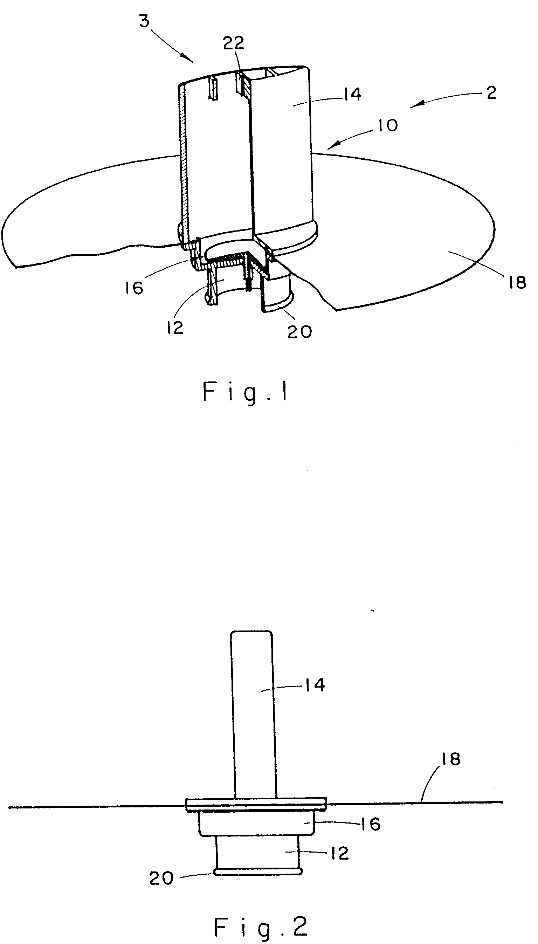

The mouth-to-mouth resuscitator shown in Figs. 1-4 comprises a vertically extending hollow body generally designated **10**, consisting of a first top portion **12**, a second, lower portion **14**, separated from one another by a valve assembly, generally designated **16**, and comprising also a laterally extending pliable sheet **18** intended to isolate the mouth area of the victim from the mouth of the rescuer.

The first portion **12** serves as a mouth piece, and has a circular cross-sectional shape and an external annular ridge **20** at its upper end. This structure of the mouth piece allows the performance of a mouth-to-mouth resuscitation virtually independent of the relative position of the rescuer and the victim, namely the rescuer can attach his mouth when situated behind the victim's head or when positioned at the victim's side. Furthermore, the annular ridge allows firm grip of the mouth piece by the rescuer's mouth.

The second portion **14** is adapted for insertion into a victim's mouth while depressing its tongue, and has reinforcing ribs **22** at its lower end. The second portion may have an elliptical cross-sectional shape as in the embodiment shown herein, it may have an ovoid cross-sectional shape or generally any suitable cross-sectional shape; furthermore it may be straight as in the embodiment shown herein or may be slightly bent.

As can be seen in the figures, the valve assembly **16** is distanced from the bottom end of the second portion **14** and thus the space inside second portion **14** serves as a buffer zone. It should be noted that in view of the one-way direction of air passage in the resuscitator, this buffer zone is generally filled with air exhaled from the rescuer. Thus, if for some reason, during resuscitation, some exhaled air, saliva, etc., of the victim is emitted into the space inside second portion **14**, it will still not reach the rescuer, even in the unlikely event of a burst in the valve assembly, as it will be absorbed inside this buffer zone.

The valve assembly, which consists of a valve seat **24** and a valve member **26**, and its manner of operation will be described in more details below.

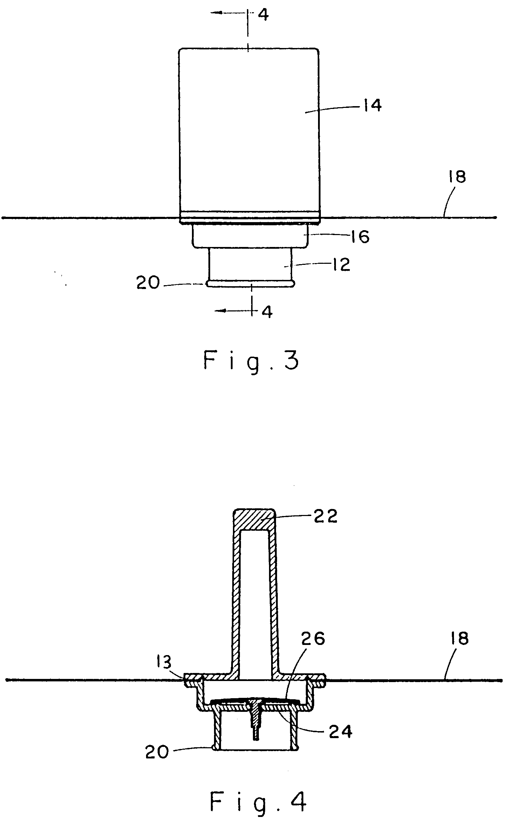

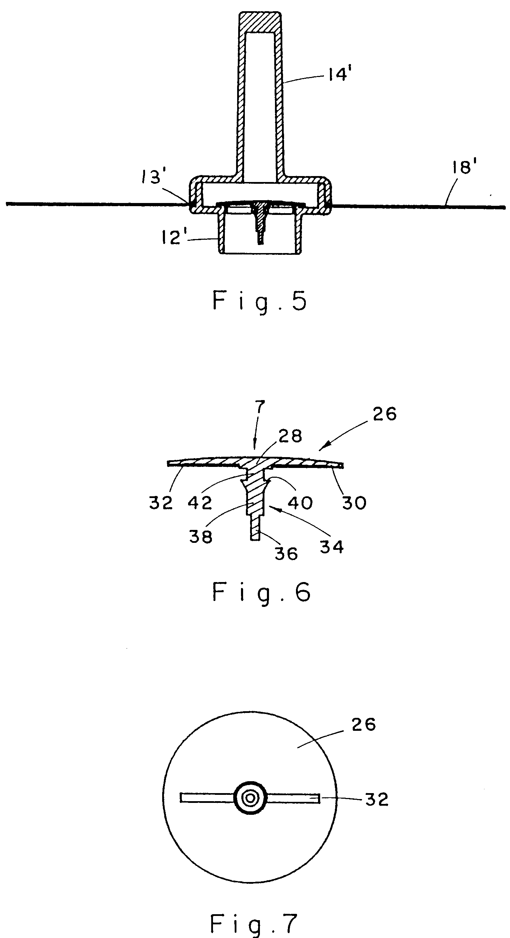

In the embodiment of Figs. 1-4, the first portion **12** with the valve assembly **16** is formed as a separate component than that forming the second partion **14**. These two components and the two are firmly attached to one another, whereby they sandwich and firmly hold at their point of attachment **13** (see Fig. 4) the rims of the central hole in sheet **18**. The attachment between these components is performed by any of a number of means generally known *per se.* Against this, in the embodiment of Figs. 5-9, the two respective components defining the first and second portions, designated in Fig. 5 as **12**' and **14**' respectively, are pressure engaged into one another, as can be seen in Fig. 5, whereby they firmly hold at the point of attachment, **13**', the rim of the central hole in pliable sheet **18**'. Pressure engagement of the different components may at times be advantageous since the different components can be disassembled, cleaned and sterilized separately for further use. Additionally, components **14**' and **18**' may be made to be disposable while component **12**' may be reusable.

It should be noted that pliable sheet **18**, rather than being firmly held between the aforementioned two components, may also be supplied as a separate component and then fitted by the user around the hollow body prior to use.

Apart from the abovenoted differences between the embodiment of Figs. 1-4 and the embodiment of Figs. 5-9, the two are essentially identical in all other features as well as in their manner of operation.

The valve assembly will now be described with reference to Figs. 6-9.

The valve member **26** shown in Figs. 6 and 7 is made of a flexible material such as silicone rubber and has generally the shape of a disk with thicker center **28** and narrow peripheral **30** portions. The valve member has two radial ribs **32** on its upper face. As a result of the presence of these ribs, the valve member has a zone of varying degree of rigidity (or flexibility) in its circumference, and consequently the transition time between an open and a closed state of the valve becomes more rapid. Extending normal from the valve member's upper face and integral therewith is an anchoring leg generally designated **34** having a first, terminal narrow cylindrical portion **36**, a middle, partially cylindrical and partially chopped conical portion **38** having shoulders **40** and a second, cylindrical portion **42** adjacent the disk.

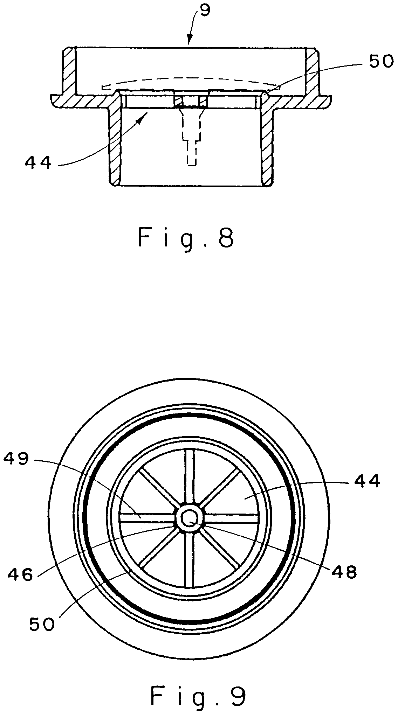

The valve seat **24**, seen in Figs. 8 and 9 has a central portion, generally designated **44** comprising in its middle a hub-like member **46** having a central bore **48** of essentially the same diameter as the second cylindrical portion **42** and has radial spokes **49** extending from the hub-like member. One important function of the spokes is to provide a rigid support for the valve member, *inter alia* to avoid its reversal in case of back pressure, e.g. when the victim coughs. The voids between the spokes provide for air passage through the valve seat. The valve seat further has a peripheral annular ridge **50** protruding downwards from the bottom face thereof.

In order to anchor the valve member in the valve seat, the anchoring leg **34** is inserted into bore **48** until shoulders **40** pass past the bore when the shoulders **40** becomes juxtaposed to the upper surface of the valve seat whereby the valve member becomes anchored in the valve seat. In this anchoring position, shown by dotted lines in Fig. 7, the valve member is generally upwardly biased, owing to its elasticity, and thus the peripheral portions thereof press against the annular ridge and form an air-tight seal. When air is exhaled by the rescuer, pressure rises in the first portion and when it reaches a level exceeding the biasing force, the peripheral portions of the valve member flex downward and consequently air is allowed to flow. When pressure is reduced, below said level, the tight seal is formed again and air flow is blocked. A valve assembly of this kind thus ensures that there is virtually no risk of any air or other fluids exhaled from the victim passing to the first member and from there to the rescuer.

It should be noted that in addition to the above described manner of upwardly biasing the valve member, it is also possible to ensure such a bias by means of a spring held in a suitable housing and pressing on a floating valve member to form an airtight seal with the valve's seat periphery. It will no doubt be appreciated that various other embodiments of valve assembly can be envisaged having feature as defined above.

## Claims

1. A mouth-to-mouth resuscitator device for use by a rescuer in order to force air into a victim while avoiding direct mouth-to-mouth contact between the rescuer and the victim, said device comprising:
a vertically extending hollow open-ended body (10) adapted to transmit air exhaled from the rescuer to the victim and having a first, top (12) and a second, bottom (14) portions, the first portion (12) has an essentially circular mouth piece at its upper end, and the second portion (14) is adapted to be inserted into the victim's mouth;
a one-way valve assembly (16) contained in said hollow body (10) and dividing it into said first (12) and second (14) portions and permitting air passage only from the first portion (12) into the second portion (14), the valve assembly (16) comprising a rigid valve seat (24) and a flexible valve member (26) below the valve seat (24), the valve seat (24) having a central portion (44) allowing free air passage, and the valve member (26) being essentially disk-shaped and being upwardly biased against the valve seat (24) so that peripheral portions of the valve member (26) presses against peripheral portions of the valve seat to form an air-tight contact; and
a pliable sheet (18) extending laterally from around said hollow body (10) from about the zone thereof being between said first (12) and said second (14) portions of said sheet (18), separating between the mouth area of the victim and the mouth of the rescuer.

2. A resuscitator according to Claim 1, wherein the second portion (14) is sufficiently long to serve as a buffer zone preventing solid microparticles or aerosol particles coming from the victim to reach the valve assembly.

3. A resuscitator according to Claim 1, wherein:
the central portion (44) of the valve seat (24) comprises spokes (49) radially extending from a centrally bored, hub-like member (46) in the middle of said central portion and comprising an annular ridge (50) protruding downwardly from a bottom face of the valve seat (24) at the periphery thereof;
the valve member (26) is made of an elastic material and has a diameter somewhat larger than that of the annular ridge (50), the valve member (26) having a central anchoring leg (34) adapted to be inserted in the bore (48) in said hub-like member (46) and brought into a locking position in which the valve member (26) is anchored in said valve assembly (16); and
in said locking position a peripheral portion (30) of the valve member (26) is biased against the peripheral annular ridge (50) of the valve seat (24) forming with said annular ridge (50) an airtight seal.
